(19) Europäisches Patentamt European Patent Office Office européen des brevets

(11) **EP 3 832 309 B1**

(12) **EUROPEAN PATENT SPECIFICATION**

(45) Date of publication and mention of the grant of the patent:
**01.11.2023 Bulletin 2023/44**

(21) Application number: **19844633.8**

(22) Date of filing: **24.07.2019**

(51) International Patent Classification (IPC):
**G01N 33/574** *(2006.01)*

(52) Cooperative Patent Classification (CPC):
**G01N 33/57407; G01N 33/57415; G01N 33/6887;**
G01N 2333/51

(86) International application number:
**PCT/KR2019/009139**

(87) International publication number:
**WO 2020/027484 (06.02.2020 Gazette 2020/06)**

(54) **COMPOSITION FOR DIAGNOSIS OF BONE METASTASIS OF CANCER AND KIT COMPRISING SAME**

ZUSAMMENSETZUNG ZUR DIAGNOSE VON KNOCHENMETASTASEN VON KREBS UND KIT MIT DIESER ZUSAMMENSETZUNG

COMPOSITION POUR LE DIAGNOSTIC DE MÉTASTASES OSSEUSES DE CANCER ET KIT LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2018 KR 20180090914**

(43) Date of publication of application:
**09.06.2021 Bulletin 2021/23**

(73) Proprietor: **Cytogen, Inc.**
**Seoul 05854 (KR)**

(72) Inventor: **CHO, Sun Wook**
**Seoul 06293 (KR)**

(74) Representative: **Kraus & Weisert**
**Patentanwälte PartGmbB**
**Thomas-Wimmer-Ring 15**
**80539 München (DE)**

(56) References cited:
**JP-B2- 2 845 347         US-A- 6 004 765**
**US-A1- 2003 054 985**

- **DAVIES S R ET AL: "OSTEOCALCIN AS A PROGNOSTIC INDICATOR FOR BONE METASTASIS IN DUCTAL BREAST CANCER", CALCIFIED TISSUE INTERNATIONAL, vol. 83, no. 1, 1 July 2008 (2008-07-01), pages 27-27, XP055835305,**
- **GAO Y ET AL: "Predictive value of osteocalcin in bone metastatic differentiated thyroid carcinoma", CLINICAL BIOCHEMISTRY, ELSEVIER INC, US, CA, vol. 43, no. 3, 1 February 2010 (2010-02-01), pages 291-295, XP026864930, ISSN: 0009-9120 [retrieved on 2009-09-02]**
- **DI PALMA F ET AL: "Physiological strains remodel extracellular matrix and cell-cell adhesion in osteoblastic cells cultured on alumina-coated titanium alloy", BIOMATERIALS, ELSEVIER, AMSTERDAM, NL, vol. 25, no. 13, 1 June 2004 (2004-06-01), pages 2565-2575, XP004486291, ISSN: 0142-9612, DOI: 10.1016/J.BIOMATERIALS.2003.09.026**
- **MUGURUMA YUKARI ET AL: "In Situ Visualization of Human MSC Engraftment in Bone Marrow: Integration into Murine Microenvironment and Interaction with Human HSC", BLOOD, AMERICAN SOCIETY OF HEMATOLOGY, US, vol. 104, no. 11, 16 November 2004 (2004-11-16), page 1283, XP086651341, ISSN: 0006-4971, DOI: 10.1182/BLOOD.V104.11.1283.1283**

EP 3 832 309 B1

- COLEMAN, R.E. et al.: "Osteocalcin: a potential marker of metastatic bone disease and response to treatment", European Journal of Cancer and Clinical Oncology, vol. 24, no. 7, 1 July 1988 (1988-07-01), pages 1211-1217, XP026207172, ISSN: 0277-5379, DOI: 10.1016/0277-5379(88)90130-7
- ARAI, Y. et al.: "Osteocalcin: is it a useful marker of bone metastasis and response to treatment in advanced prostate cancer?", The Prostate, vol. 20, no. 3, 1 January 1992 (1992-01-01), pages 169-177, XP055684339, ISSN: 0270-4137, DOI: 10.1002/pros.2990200302

**Description**

[Technical Field]

The present invention relates to the in vitro use of a composition for diagnosing bone metastasis of cancer and the in vitro use of a kit comprising the same.

[Background Art]

Metastatic bone cancer occurs while malignant tumors destroy bone tissue. Once metastasis bone cancer is diagnosed, the prognosis thereof becomes rapidly worse in many cases regardless of the type of cancer. The main reason for this is that diagnosis of metastatic bone cancer using current imaging tests is very late. In fact, in breast cancer that readily metastasizes to bone, "bone scanning" is performed every 6 to 12 months for prevention and early diagnosis of bone metastasis while hormone suppression therapy is usually performed for 5 years even after initial treatment. However, even when bone metastasis is diagnosed by bone scanning without symptoms such as pain, fracture or nerve compression, bone destruction has already progressed in most cases, and thus the response thereof to treatment is poor.

In general, metastatic bone cancer proceeds in the order of a resting stage, a microscopic bone metastasis stage and a clinical bone metastasis stage.

In the resting phase, cancer cells exist only in osteocytes, and no interaction occurs between cancer cells and osteocytes. However, in the microscopic bone metastasis stage, cancer cells interact with osteoclasts, and cell proliferation occurs. In the clinical bone metastasis stage, bone tissue is destroyed by cancer cells and osteoclasts, and symptoms such as pain and fracture are found.

DAVIES S R ET AL: "OSTEOCALCIN AS A PROGNOSTIC INDICATOR FOR BONE METASTASIS IN DUCTAL BREAST CANCER", CALCIFIED TISSUE INTERNATIONAL, vol. 83, no. 1, 1 July 2008 (2008-07-01), pages 27- 27, discloses the use of osteocalcin as a marker for bone metastasis of breast cancer.

GAO Y ET AL: "Predictive value of osteocalcin in bone metastatic differentiated thyroid carcinoma", CLINICAL BIOCHEMISTRY, ELSEVIER INC, US, CA, vol. 43, no. 3, 1 February 2010 (2010-02-01), pages 291 -295, discloses the use of osteocalcin as a marker for bone metastasis of thyroid carcinoma.

DI PALMA F ET AL: "Physiological strains remodel extracellular matrix and cell-cell adhesion in osteoblastic cells cultured on alumina-coated titanium alloy", BIOMATERIALS, ELSEVIER, AMSTERDAM, NL, vol. 25, no. 13, 1 June 2004 ( 2004-06-01), pages 2565-2575, discloses compositions and kits comprising antibodies for osteocalcin and primers for RT-PCR of N-cadherin which are suitable for the purpose of determining bone metastasis.

MUGURUMA YUKARI ET AL: "In Situ Visualization of Human MSC Engraftment in Bone Marrow: Integration into Murine Microenvironment and Interaction with Human HSC", BLOOD, AMERICAN SOCIETY OF HEMATOLOGY, US, vol. 104, no. 11, 16 November 2004 (2004-11-16), page 1283, discloses compositions comprising agents that a differentially fluorescently labeled antibodies that allow for a multicolor immunofluorescent staining of osteocalcin and N-cadherin which are suitable for determining bone metastasis.

Diagnosis of bone metastasis of cancer using conventional imaging tests was possible only in this clinical bone metastasis stage, and in this case, there were limitations in that since the tumor has already progressed, effective treatment was difficult, and diagnosis was possible after the morphological change of bone tissue occurred. Accordingly, there is increasing need for a diagnostic composition, a kit, or a method for providing information for diagnosis, effective treatment through early diagnosis in the microscopic bone metastasis stage before bone tissue is destroyed.

[Disclosure]

[Technical Problem]

An object of the present invention is to provide an in vitro use of a diagnostic composition and an in vitro use of a kit capable of diagnosing bone metastasis of cancer in an early stage.

Another object of the present invention is to provide a method of providing information for diagnosis of bone metastasis of cancer.

[Technical Solution]

    1. In vitro use of a composition comprising an agent for detecting cells released into blood expressing osteocalcin for diagnosing bone metastasis of cancer.

2. The use of 1, wherein the composition further comprises an agent for detecting cells released into blood expressing N-cadherin.

3. The use of 1, wherein the agent for detecting cells released into blood expressing osteocalcin is at least one selected from the group consisting of antibodies, aptamers, DNA, RNA, proteins, and polypeptides.

4. The use of 2, wherein the agent for detecting cells released into blood expressing osteocalcin and the agent for detecting blood-derived cells expressing N-cadherin are each independently at least one selected from the group consisting of antibodies, aptamers, DNA, RNA, proteins, and polypeptides.

5. The use of 1, wherein the cancer is at least one selected from among liver cancer, lung cancer, bladder cancer, stomach cancer, breast cancer, uterine cancer, colorectal cancer, colon cancer, blood cancer, ovarian cancer, prostate cancer, pancreatic cancer, spleen cancer, testicular cancer, thymus cancer, brain cancer, esophageal cancer, kidney cancer, biliary tract cancer, thyroid cancer, or skin cancer.

6. The use of 1, wherein the cancer is breast cancer or thyroid cancer.

7. In vitro use of a kit for diagnosing bone metastasis of cancer, the kit comprising the composition used according to any one of 1 to 6.

8. A method of providing information for diagnosis of bone metastasis of cancer, the method comprising a step of detecting cells released into blood expressing osteocalcin in a sample isolated from a subject to be diagnosed.

9. The method of 8, further comprising a step of detecting cells released into blood expressing N-cadherin in the sample.

10. The method of 8, wherein the cancer is at least one selected from among liver cancer, lung cancer, bladder cancer, stomach cancer, breast cancer, uterine cancer, colorectal cancer, colon cancer, blood cancer, ovarian cancer, prostate cancer, pancreatic cancer, spleen cancer, testicular cancer, thymus cancer, brain cancer, esophageal cancer, kidney cancer, biliary tract cancer, thyroid cancer, or skin cancer.

11. The method of 8, wherein the cancer is breast cancer or thyroid cancer.

[Advantageous Effects]

According to the present invention, it is possible to diagnose bone metastasis of cancer by a simpler method than bone scanning, and to significantly increase the survival rate of a patient by dramatically advancing the time when bone metastasis of cancer is diagnosed.

[Description of Drawings]

FIG. 1 is a view showing an example of a method of providing information for early diagnosis of bone metastasis of bone according to the present invention, and shows the results of quantifying circulating osteocalcin-positive (osteocalcin+) or osteocalcin-positive/N-cadherin-positive (osteocalcin+/N-cadherin+) cells in cancer patient blood by flow cytometry.

FIG. 2 shows the preparation of a bone metastatic breast cancer mouse model and the results of bioluminescence imaging (BLI) and histopathological examination in the mouse model at 2 weeks, 3 weeks and 5 weeks.

FIG. 3(A) is a graph showing that bone metastasis of cancer can be diagnosed from week 5 in BLI, but circulating osteocalcin-positive cells can be diagnosed from week 2 to 3, and FIG. 3(B) shows that the tumor size and the number (%) of circulating osteocalcin-positive cells are proportional to each other.

FIG. 4 shows the results of injecting a breast cancer cell line into the left ventricle of a mouse to prepare a mouse model that is most similar to an actual bone metastatic breast cancer patient, performing bioluminescence imaging (BLI) on varying days, and analyzing calculating osteocalcin-positive cells by flow cytometry on varying days.

FIG. 5 shows the results of preparing mouse models using different mouse species and breast cancer cells associated with the mouse species, dividing the mice into a bone metastasis group and a control group, and analyzing calculating osteocalcin-positive cells by flow cytometry.

FIG. 6 shows the results of measuring the number of calculating osteocalcin-positive cells in metastatic breast cancer patients with or without bone metastasis.

FIG. 7 shows the results of analyzing calculating osteocalcin-positive cells in a stable group without disease progression and a progressive group with disease progression depending on whether metastatic bone disease is active or inactive.

FIG. 8 shows the results of determining an optimal cut-off value capable of predicting the progression of metastatic bone disease, dividing metastatic bone cancer patients into two group according to the cut-off value, and performing progression-free survival (PFS) analysis.

FIG. 9(A) shows the results of analyzing calculating osteocalcin-positive cells in metastatic thyroid cancer patients divided into an inactive group and an active group according to whether bone metastasis is active or inactive at the time of cOC% analysis. FIG. 9(B) shows the results of analyzing calculating osteocalcin-positive cells in a stable group without disease progression and a progressive group with disease progression.

[Mode for Invention]

Hereinafter, the present invention will be described in detail.

As used herein, the term "diagnosis" means identifying the presence or characteristics of a pathological condition. With regard to the purpose of the present invention, the term "diagnosis" may mean identifying the presence of bone metastasis of cancer, or confirming whether or not bone metastasis of cancer is progressing or severe.

As used herein, the term "diagnosis" may encompass determining the susceptibility of a subject to a certain disease or disorder, determining whether a subject is affected with a certain or disorder, determining the prognosis of a subject affected with a certain or disorder, or therametrics (e.g., monitoring the state of a subject to provide information on therapeutic efficacy).

The present invention is directed to the in vitro use of a composition comprising an agent for detecting cells released into blood expressing osteocalcin diagnosing bone metastasis of cancer.

The present inventors have found that osteocalcin-positive cells are released into blood in the microscopic bone metastasis stage, which is an early stage of bone metastasis of cancer, to form circulating osteocalcin-positive cells, and bone metastasis of cancer may be diagnosed in an early stage by detecting the circulating osteocalcin-positive cells, thereby completing the present invention.

The agent for detecting cells released into blood expressing osteocalcin is not limited as long as it may detect osteocalcin, but examples thereof include antibodies, aptamers, DNA, RNA, proteins, and polypeptides, which target osteocalcin, mRNA which is translated to osteocalcin, DNA which is transcribed into the mRNA, or DNA having a sequence complementary thereto.

When the composition comprises the agent for detecting cells released into blood expressing osteocalcin, it may be used for diagnosis of bone metastasis of cancer. However, for more accurate diagnosis of bone metastasis of cancer, the composition may further comprise an agent for detecting cells released into blood expressing N-cadherin.

The agent for detecting cells released into blood expressing N-cadherin is not limited as long as it may detect N-cadherin, but examples thereof include antibodies, aptamers, DNA, RNA, proteins, and polypeptides, which target N-cadherin, mRNA which is translated to N-cadherin, DNA which is transcribed into the mRNA, or DNA having a sequence complementary thereto.

As used herein, the term "antibody" refers to a protein molecule specific to an antigenic site. With regard to the purposes of the present invention, the term "antibody" refers to an antibody that binds specifically to the marker protein osteocalcin or N-cadherin, and may include all monoclonal antibodies, polyclonal antibodies and recombinant antibodies.

The monoclonal antibody may be produced using a hybridoma method well known in the art or a phage antibody library technique, but is not limited thereto.

The polyclonal antibody may be produced by a method well known in the art, which comprises injecting the protein antigen into an animal, collecting blood from the animal, and isolating serum containing the antibody. This polyclonal antibody may be produced from any animal species hosts such as goats, rabbits, sheep, monkeys, horses, pigs, cattle, or dogs, but is not limited thereto.

In addition, the antibodies of the present application may also include special antibodies such as chimeric antibodies, humanized antibodies, and human antibodies.

The "peptide" has the advantage of high binding affinity for the target material, and does not denature even during thermal/chemical treatment. In addition, since the peptide has a small molecular size, it may be attached to another protein and used as a fusion protein. Specifically, since the peptide may be used attached to a polymer protein chain, it may be used in a diagnostic kit and as a drug delivery material.

The "aptamer" refers to a kind of polynucleotide consisting of a special kind of single-stranded nucleic acid (DNA, RNA or modified nucleic acid) capable of binding to a target molecule with high affinity and specificity while having a stable three-dimensional structure. As described above, the aptamer is composed of a polynucleotide which may bind specifically to an antigenic substance in the same manner as an antibody, and at the same time, is more stable than a protein, has a simple structure, and is easy to synthesize. Thus, the aptamer may be used instead of an antibody.

In addition, the cancer may be at least one selected from among liver cancer, lung cancer, bladder cancer, stomach cancer, breast cancer, uterine cancer, colorectal cancer, colon cancer, blood cancer, ovarian cancer, prostate cancer, pancreatic cancer, spleen cancer, testicular cancer, thymus cancer, brain cancer, esophageal cancer, kidney cancer, biliary tract cancer, thyroid cancer, or skin cancer. Preferably, the cancer may be at least one selected from among breast cancer, thyroid cancer or prostate cancer. More preferably, the cancer may be breast cancer or thyroid cancer, but is not limited thereto.

The present invention is also directed to the in vitro use of a kit for diagnosing bone metastasis of cancer, the kit comprising the above composition.

The kit for use of the present invention may comprise an antibody that specifically binds to a marker component, a secondary antibody conjugated with a label that develops color by reaction with a substrate, a chromogenic substrate solution for reaction with the label, and a washing solution, and an enzyme reaction stop solution, and may be made of a plurality of separate packaging or compartments including the reagent components used, but is not limited thereto.

The kit for use of the present invention may comprise not only an agent capable of measuring the expression level of osteocalcin or N-cadherin in a patient sample, but also one or more compositions, solutions, or devices suitable for analysis of the expression level. For example, the kit may comprise a substrate, an appropriate buffer solution, a secondary antibody conjugated with a detection label, and a chromogenic substrate, for immunological detection of an antibody, but is not limited thereto.

As a specific example, the kit may be a kit comprising essential elements necessary for performing ELISA in order to implement various ELISA methods such as an ELISA kit, a sandwich ELISA, and the like. This ELISA kit comprises an antibody specific for the protein. The antibody may be a monoclonal antibody, a polyclonal antibody or a recombinant antibody, which has high specificity and affinity for osteocalcin or N-cadherin protein and has little cross-reactivity with other proteins. In addition, the ELISA kit may comprise an antibody specific for a control protein. In addition, the ELISA kit may comprise reagents capable of detecting bound antibodies, for example, labeled secondary antibodies, chromophores, enzymes, and other substances capable of binding to their substrates or antibodies, but is not limited thereto.

In addition, the kit may be a kit for implementing Western blot assay, immunoprecipitation assay, complement fixation assay, flow cytometry, or protein chip assay, and may further comprise additional components suitable for each assay method. Through these assay methods, it is possible to diagnose bone metastasis of cancer by comparing the amount of antigen-antibody complex formed.

The present invention is also directed to a method of providing information for diagnosis of bone metastasis of cancer, the method comprising a step of detecting cells released into blood expressing osteocalcin in a sample isolated from a subject to be diagnosed.

The subject to be diagnosed is an animal that currently has cancer or has a history of cancer, and the animal may be a mammal including a human being.

The sample is isolated from the subject to be diagnosed, and examples thereof include, but are not limited to, tissue, cells, whole blood, plasma, serum, blood, or cerebrospinal fluid. Specifically, the sample may be blood, plasma, serum, or the like. More specifically, the sample may be peripheral blood mononuclear cells.

The detection is performed to detect osteocalcin, and may be performed by treating the sample with a composition for diagnosing bone metastasis of cancer comprising an agent for detecting cells released into blood expressing osteocalcin.

The method may further comprise detecting cells released into blood expressing N-cadherin in the sample. In this case, the detection may be performed by treating the sample with a composition for diagnosing bone metastasis of cancer comprising an agent for detecting cells released into blood expressing osteocalcin and an agent for detecting cells released into blood expressing N-cadherin.

In terms of further increasing the accuracy of diagnosis, both osteocalcin and N-cadherin may be detected, but are not limited thereto.

The scope of the agent for detecting cells released into blood expressing osteocalcin or the agent for detecting cells released into blood expressing N-cadherin is as described above.

The scope of the cancer is as described above.

The method of the present invention is intended to detect cells released into blood expressing osteocalcin in a sample isolated from a subject to be diagnosed, or to detect cells released into blood expressing osteocalcin and N-cadherin, and the detection may be performed using, for example, an antibody, peptide, aptamer or protein specific for osteocalcin or N-cadherin, but is not limited thereto.

As a specific example, the method for detection may be performed using an antibody specific for osteocalcin or N-cadherin by a method selected from the group consisting of enzyme-linked immunosorbent assay (ELISA), radioimmunoassay (RIA), sandwich assay, Western blotting, immunoprecipitation, immunohistochemical staining, flow cytometry, fluorescence activated cell sorting (FACS), enzyme substrate colorimetric assay, antigen-antibody aggregation, and protein chip assay, but is not limited thereto.

As an additional method, it is possible to a technique of detecting two fluorescent substances, which are at close distance, at one fluorescence wavelength using a fluorescence resonance energy transfer that creates new color development by transferring resonance energy. In addition, in order to increase the objectivity and reproducibility of tests other than flow cytometry, it is possible to use a technique comprising labeling cells with an antibody, filtering out cells having a size of 10 $\mu$m or less through a mesh filter, plating the cells on a slide, automatically scanning the slide, and then quantifying the number of positive cells by artificial intelligence.

In addition, the method of the present invention may further comprise a step of determining that the cancer has metastasized to the bone, when cells released into blood expressing osteocalcin are detected.

When cells released into blood expressing osteocalcin are detected in the sample from the subject to be diagnosed, it may be determined that the cancer has metastasized to the bone, and as the amount of osteocalcin detected increases, it may be determined that the bone metastasis stage of the cancer has further progressed.

When the method of the present invention further comprises the step of detecting cells released into blood expressing N-cadherin, if N-cadherin is also detected, bone metastasis of cancer may be diagnosed with higher accuracy.

If necessary, the method of the present invention may further comprise a step of treating the sample, isolated from the subject to be diagnosed, with vitamin K.

The vitamin K may be vitamin K1 or vitamin K2.

Treatment of the sample with vitamin K may γ-carboxylate osteocalcin and increase and stabilize the expression of osteocalcin, so that osteocalcin may be more easily detected.

The treatment concentration of vitamin K is not particularly limited, and may be, for example, $10^{-5}$ M to $10^{-7}$ M. Specifically, the treatment concentration of each of vitamin K1 and vitamin K2 may be $10^{-5}$ M to $10^{-7}$ M. At the above-described treatment concentration, the osteocalcin expression effect of circulating osteocalcin-positive cells may be maximized.

Further described but not claimed is a method of diagnosing bone metastasis of cancer by detecting osteocalcin in a sample isolated from a subject to be diagnosed.

The scope of the sample is as described above.

The detection may be performed by treating the sample with an agent for detecting osteocalcin.

The agent for detecting osteocalcin is as described above.

When osteocalcin is detected, it may be determined that the cancer has metastasized to the bone. For a more specific example, bone metastasis of cancer may be diagnosed by measuring the number (%) of circulating osteocalcin-positive cells in the sample. Specifically, bone metastasis of cancer may be diagnosed by comparing the number of circulating osteocalcin-positive cells in a patient having bone metastasis with the number of circulating osteocalcin-positive cells in a patient having no bone metastasis, but is not limited thereto.

In addition, the method may further comprise a step of detecting N-cadherin in the sample.

This step may be performed by treating the sample with an agent for detecting N-cadherin, and the scope of the agent is as described above.

When N-cadherin is detected, bone metastasis of cancer may be diagnosed with higher accuracy. Specifically, it is possible to detect circulating osteocalcin-positive cells in the sample, and then detect circulating N-cadherin-positive cells, but is not limited thereto.

Hereinafter, the present invention will be described in detail with reference to examples.

Examples

1. Isolation of Peripheral Blood Mononuclear Cells and Flow Cytometry

**(1) Experimental Method**

For flow cytometry of circulating osteocalcin-positive cells in blood samples isolated from cancer patients, each sample was collected via liquid biopsy, and peripheral blood mononuclear cells were isolated from the sample. 4 cc of the blood sample collected from the patient to be diagnosed was placed in a heparin tube containing Ficoll (Pharmacia) and was centrifuged at a speed of 1800 rpm at a temperature of 4°C for 20 minutes to separate the blood into plasma, a mononuclear cell layer, Ficoll and an RBC layer. At this time, only the mononuclear cell layer (second layer) at the interface between the separated plasma and Ficoll was isolated carefully and transferred into a fresh tube. Next, the mononuclear cells were washed with PBS solution and then centrifuged at a speed of 2000 rpm at a temperature of 4°C for 5 minutes, and only cleanly washed mononuclear cells were collected.

$10^6$ washed mononuclear cells were placed in 500 μl of FBS-containing buffer and stained by incubation with anti-CD15-FITC (5 μl), anti-CD34-PE-Cy7 (5 μl), anti-osteocalcin-APC (3 μl) and anti-N-cadherin-PE (5 μl) antibodies at room temperature for 30 minutes. (Information on antibodies used: a-CD15 FITC (#555401,BD), a-CD34 PE-Cy7 (#34881, BD), a-osteocalcin APC (#SC-365797-AF647, Santa Cruz), and a-N-cadherin PE (#561554, BD) were purchased and used).

After completion of staining, non-specifically bound and unbound antibodies were washed out by adding 4 ml of FBS-containing buffer, and antibody-labeled mononuclear cells were obtained by concentration at a speed of 1500 rpm at a temperature of 4°C for 5 minutes. The above-described cancer patient blood samples were obtained according to regulations under approval of the IRB of the Clinical Research Center of Seoul National University Hospital for use in clinical research in the present invention.

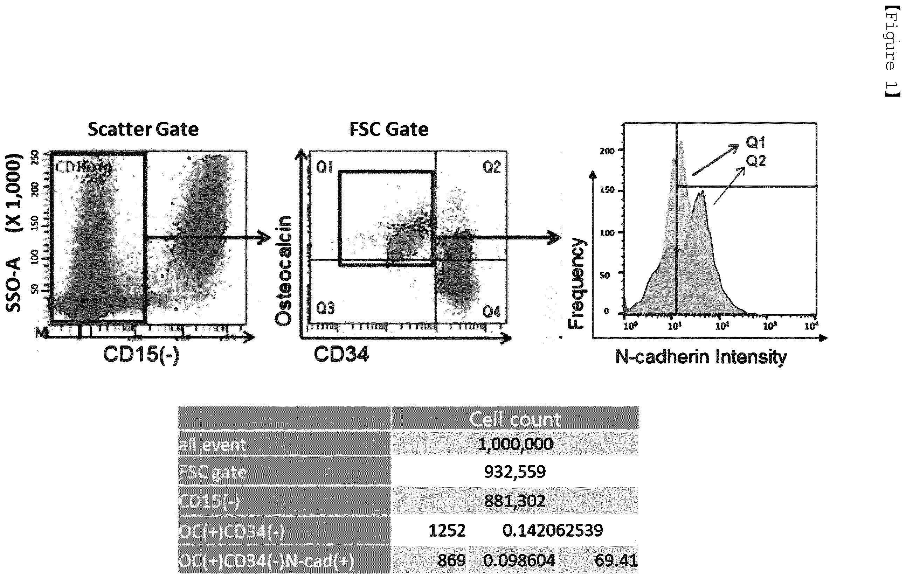

The stained mononuclear cells were analyzed by flow cytometry (BD FACSCanto II). The mononuclear cells stained with the above-listed antibodies were analyzed at the single-cell level, and first sorted into CD15- cell populations, and re-sorted into CD34- and osteocalcin+ cell populations. The number of the cells was quantified, and circulating osteocalcin-positive cells were analyzed. In addition, the number of N-cadherin+ cells in the corresponding population was analyzed, and finally, circulating CD15-/CD34-/osteocalcin+/N-cadherin+ cells specific to metastatic bone cancer were quantified using Equation 1 below. Analyses of circulating osteocalcin-positive cells in samples obtained from a total of 108 cancer patients (98 breast cancer cells and 10 thyroid cancer patients) were performed, and the results of representative analysis are shown in FIG. 1.

```
[Equation 1]

Circulating osteocalcin-positive cells (%) = {(number

of  CD15-/CD34-/osteocalcin+/N-cadherin+  cells)/(number  of

CD15- cells)} X 100(%)
```

**(2) Experimental Results**

In flow cytometry of the cancer patient blood sample, a total of 1,000,000 cells were analyzed, and among them, 932,559 cells excluding dead cells were sorted. Subsequently, 881,302 CD15- cells were re-sorted, from which 1,252 osteocalcin+/CD34- cells were then sorted. Among 1,252 cells, 869 N-cadherin+ cells were sorted. The results are shown in FIG. 1.

**2. Preparation of Metastatic Bone Cancer Mouse Model**

**(1) Experimental Method**

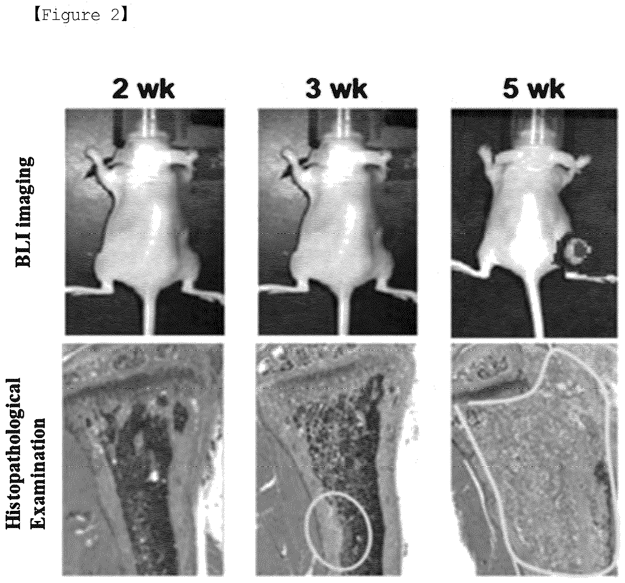

For analysis of circulating osteocalcin-positive cells in a preclinical study, a mouse model was prepared, which facilitates analysis of the bone marrow environments and circulating osteocalcin-positive cells of actual patients with metastatic bone cancer. All animal experiments were conducted in accordance with strict regulations under approval of the Seoul National University Animal Experimental Ethics Committee (SNU-170607-6-2). An ideal animal model similar to metastatic bone cancer patients was prepared by injecting a human breast cancer cell line directly into the tibia of each immunodeficient mouse (Balb/c nude mouse). Specifically, the human breast cancer cell line MDA-MB-231 was prepared in PBS (buffer injectable into the mouse body) in an amount of $1 \times 10^5$ cells/20 $\mu$l/mouse, and injected into the right tibia of each nude mouse. At 2, 3 and 5 weeks after injection of the cancer cells, formation and growth of tumors in the tibia was observed through *in vivo* imaging (Xenogen IVIS, PerkinElmer). In order to confirm substantial tumor formation at each week, the mouse was euthanized, the tumor-formed tibia was isolated, and a process of preparing a sample for histological analysis was performed. The isolated tissue was fixed by cold storage in 4% para-formaldehyde solution for 3 days, and then the muscle portion was removed from the bone which was then decalcified in 0.5M EDTA solution for about 2 weeks. (During the decalcification process, the tissue was cold-stored, and 0.5 M EDTA solution was replaced every 3 days). Then, the tissue was embedded in paraffin and sectioned, and the paraffin sections were placed on slides and stained with H&E. The results of *in vivo* imaging and tissue staining are shown in FIG. 2.

**(2) Experimental Results**

As a result of performing BLI imaging of the prepared bone metastatic breast cancer mouse model at each week, it could be confirmed that tumor formation and growth could be found even at 5 weeks. In addition, the results of histopathological examination were consistent with the results of BLI imaging. At 3 weeks, it was observed that a very small tumor was formed in the tibia, and at 5 weeks, it could be observed that a large tumor was formed in the tibia. These results suggest that a microscopic tumor is difficult to diagnose by imaging, and the tumor can be diagnosed when it has grown significantly for 5 weeks or more (FIG. 2).

**3. Analysis of Change in Circulating Osteocalcin-Positive Cells Caused by Tumor Growth**

**(1) Experimental Method**

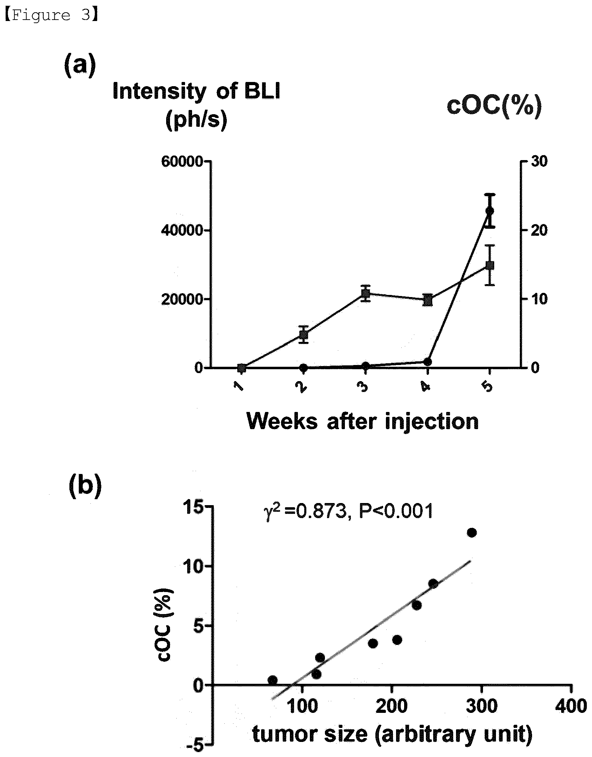

In order to analyze changes in circulating osteocalcin-positive cells depending on the tumor size in the metastatic bone cancer mouse model prepared in Example 2, blood sampling was performed. Specifically, the mouse was anesthetized and about 1 ml of whole blood was sampled from the mouse through cardiac puncture. In order to prevent blood clotting during blood collection, the inside of the syringe was coated with a heparin solution before blood sampling, and the sampled blood was added to a red blood cell lysis solution immediately after sampling and subjected to a red blood cell lysis process. The sample was left to stand at room temperature for 15 minutes, and then centrifuged at 1500 rpm for 5 minutes, and the supernatant was removed. This process was repeated until the red blood cells were completely removed, and cells from which the red blood cells have been completely removed were added to a buffer containing FBS. Thereafter, the cells were stained with anti-CD45-PE (#561087, BD) and the anti-osteocalcin antibody described in Example 1 above, and circulating CD45+/osteocalcin+ cells were analyzed by flow cytometry. The intensity of *in vivo* bioluminescence imaging (ph/s) and the number (%) of circulating osteocalcin-positive cells (cOC) as a function of the tumor size were compared. The results are shown in FIG. 3.

**(2) Experimental Results**

In the metastatic bone cancer mouse model prepared using breast cancer cells, it could be seen that circulating osteocalcin-positive cells (cOC) increased before diagnosis in the imaging technique, and that tumor cells could be detected by histopathological analysis when these cells started to form microclusters on the bone surface (see FIG. 2). It could be confirmed that bone metastasis of cancer could be diagnosed at 5 weeks in conventional BLI imaging, but could be diagnosed at 2 to 3 weeks according to the present invention (FIG. 3(A)).

In addition, in the metastatic bone cancer mouse model prepared using breast cancer cells, circulating osteocalcin-positive cells (cOC) showed a good correlation with the tumor size measured by pathological analysis that can be considered as a gold standard (FIG. 3(B)).

**4. Preparation of Microscopic Bone Metastatic Breast Cancer Mouse Model and Analysis of Circulating Osteocalcin-Positive Cells**

**(1) Experimental Method**

As a mouse model different from the bone metastasis mouse model prepared by injecting cancer cells into the tibia, a mouse model was prepared by injecting breast cancer cells directly into the left ventricle of each mouse in order to conduct a preclinical study in an environment which is most similar to an actual bone metastatic breast cancer patient. Human breast cancer MDA-MB-231 cells were injected directly into the left ventricle of each immunodeficient mouse (Balb/c nude mice) to establish a mouse model in which the cancer cells metastasized to the tibia or femur after systemic circulation.

Specifically, the human breast cancer line MDA-MB-231 was prepared in PBS (buffer injectable into the mouse body) in an amount of $1 \times 10^5$ cells/100 $\mu$l/mouse, and injected directly into the left ventricle of each nude mouse. On 3, 7, 14 and 22 days after injection of the cancer cells, formation and growth of the tumor that metastasized to the mouse bone was observed through *in vivo* imaging (Xenogen IVIS, PerkinElmer). In addition, a region of interest (ROI) was set and analyzed, and the growth of the tumor that metastasized to the mouse bone was numerically analyzed. On the above-described days, each mouse was euthanized, whole blood was sampled from the mouse, PBMCs were isolated therefrom, and circulating osteocalcin-positive cells therein were analyzed. The blood sampling and PBMC isolation process, and antibody staining for flow cytometry are as described in Example 3 above.

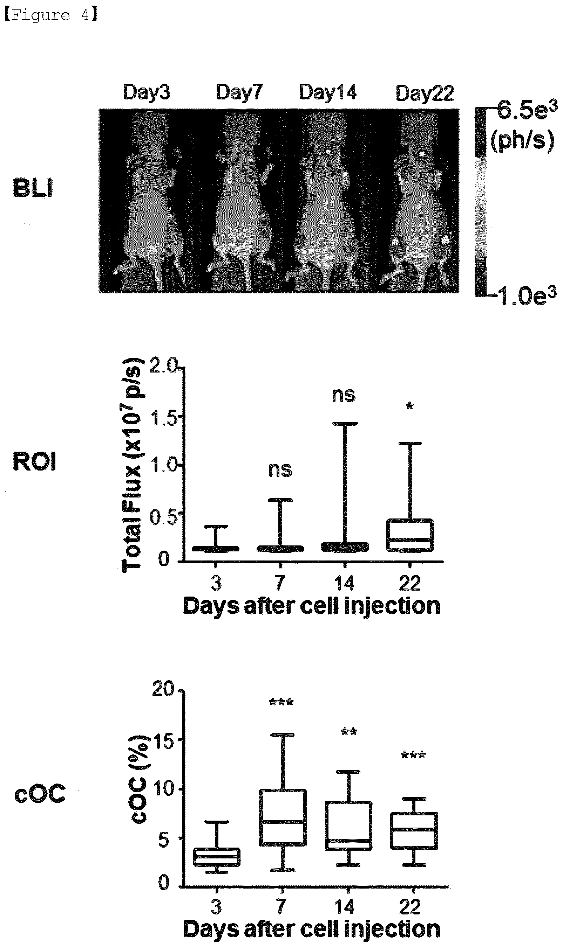

The results of *in vivo* imaging and circulating osteocalcin-positive cell are shown in FIG. 4.

**(2) Experimental Results**

As a result of preparing the microscopic bone metastatic breast cancer mouse model and performing BLI imaging of the mouse model on varying days in the early stage of bone metastasis, it could be confirmed that bone metastasis could be diagnosed on 14 days after injection of the cancer cells and that significantly analysis results for the ROI value could be obtained only on 22 days after cell injection. However, the results of analysis of circulating osteocalcin-positive cells indicated that the circulating osteocalcin-positive cells already increased on day 7 when bone metastasis could not be detected by BLI imaging. These results are consistent with the results obtained in the model prepared by injecting cancer cells directly into the tibia, and suggest that microscopic metastatic bone tumors are difficult to diagnose by BLI imaging, but early diagnosis thereof is possible by analysis of circulating osteocalcin-positive cells (FIG. 4).

**5. Analysis of Circulating Osteocalcin-Positive Cells Depending on Presence or Absence of Bone Metastasis**

**(1) Experimental Method**

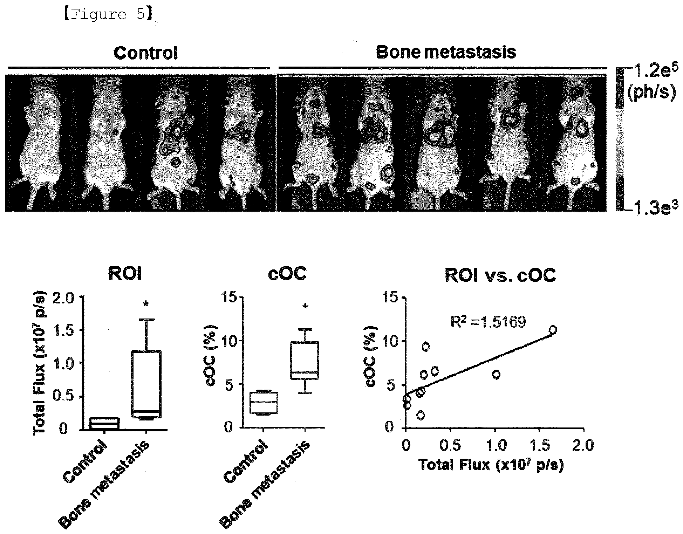

To analyze circulating osteocalcin-positive cells depending on the presence or absence of bone metastasis, the same microscopic bone metastatic breast cancer model as that in Example 4 was prepared by injecting cancer cells into the left ventricle. The mouse model was prepared using a breast cancer cell line derived from the corresponding mouse species in other mouse species, in order to demonstrate that the quantitative increase in circulating osteocalcin-positive cells is not a mouse species-specific response or a specific breast cancer cell line response. Specifically, the Balb/c mouse-derived breast cancer cell line 4T1 was prepared in PBS (buffer injectable into the mouse body) in an amount of $1 \times 10^4$ cells/100 $\mu$l/mouse, and injected directly into the left ventricle of each Balb/c mouse. On 7 days after injection of the cancer cells, the mice were divided into a group with bone metastasis and a group without bone metastasis through BLI imaging, and the intensity of luminescence between the bone metastasis group and the control group was quantified through ROI analysis. Thereafter, the mice were sacrificed, whole blood was collected therefrom, and circulating osteocalcin-positive osteocalcin cells therein were analyzed in the same manner as described in the above Example. In addition, the correlation between the results of BLI analysis and the results of circulating osteocalcin-positive cell analysis was analyzed.
The results are shown in FIG. 5.

**(2) Experimental Results**

As a result of analyzing circulating osteocalcin-positive cells in the bone metastasis group and the control group, a significant quantitative increase in circulating osteocalcin-positive cells in the bone metastasis group compared to that in the control group could be confirmed. In addition, as a result of analyzing the correlation between the result of BLI imaging and the quantitative increase in circulating osteocalcin-positive cells, it was demonstrated that the quantitative increase in circulating osteocalcin-positive cells had a significant correlation with the presence of bone metastasis. This proves that circulating osteocalcin-positive cells may serve as a marker not only for early diagnosis of microscopic bone metastatic breast cancer, but also capable of detecting the presence of bone metastasis (FIG. 5) .

**6. Clinical Study on Metastatic Breast Cancer Patients**

**(1) Experimental Method**

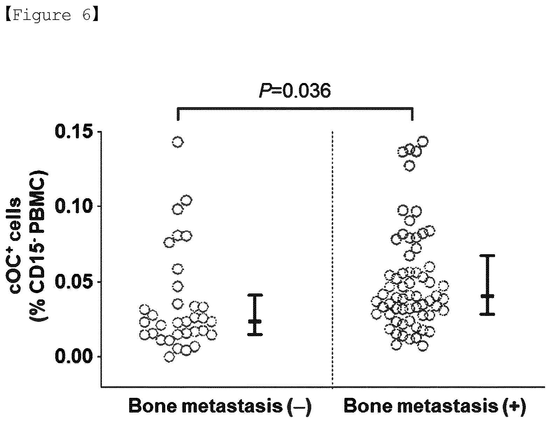

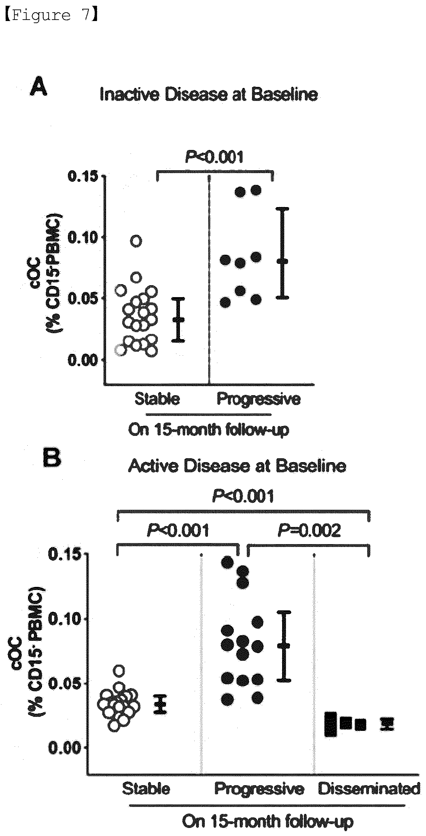

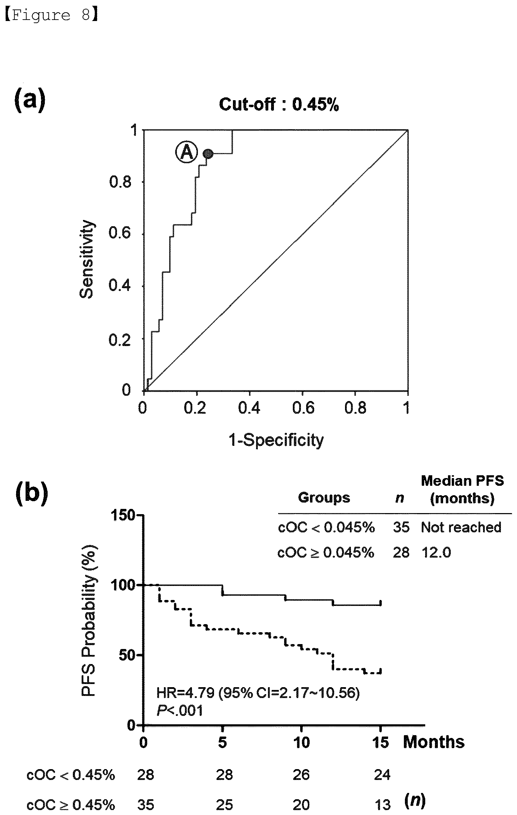

In order to evaluate whether circulating osteocalcin-positive cells present in the blood of actual patients with metastatic bone cancer have clinical diagnostic value by analyzing the circulating osteocalcin-positive cells, the number (%) of circulating osteocalcin-positive cells was measured on 96 breast cancer patients with metastatic bone cancer. Patient's blood sampling, mononuclear cell isolation and cell staining processes were performed in the same manner as described in Example 1 above, and the analysis method was also performed in the same manner. The results are shown in FIG. 6.
In order to evaluate additional clinical value, various analysis methods were performed. At the time of cOC% analysis and after continuous follow-up, the patients were divided into a group with active metastatic bone disease and a group with inactive disease and analyzed. The results are shown in FIG. 7. In addition, based on the results of the follow-up analysis, an optimal cut-off value capable of predicting the progression of metastatic bone disease was determined, and based on the cut-off value, progression-free survival (PFS probability) analysis was performed. The results are shown in FIG. 8.

**(2) Experimental Results**

1. Among 96 metastatic breast cancer patients, 63 patients with bone metastasis had significant high cOC% compared to 33 patients without bone metastasis (FIG. 6).
2. When the patients with bone metastasis were divided into an inactive group of 26 patients and an active group of 37 patients according to whether the metastatic bone disease was active or inactive at the time of cOC% analysis, there was no difference in cOC% between the two groups. However, when the patients were divided into a stable group without progression of metastatic bone disease and a progressive group with progression of metastatic bone disease after 15-month follow-up, it could be confirmed that, in both the inactive group and the active group, the baseline cOC% of the progressive group was significantly higher than that of the stable group (FIG. 7).

3. The optimal cut-off value enabling cOC% to predict the progression of the metastatic bone disease by cOC% could be determined as 0.45% (FIG. 8(A)). When the patients with metastatic bone cancer were divided into two groups based on 0.45%, it could be confirmed that there was a significant difference in progression-free survival between the two groups (FIG. 8(B)).

### 7. Clinical Study on Metastatic Thyroid Cancer Patients

#### (1) Experimental Method

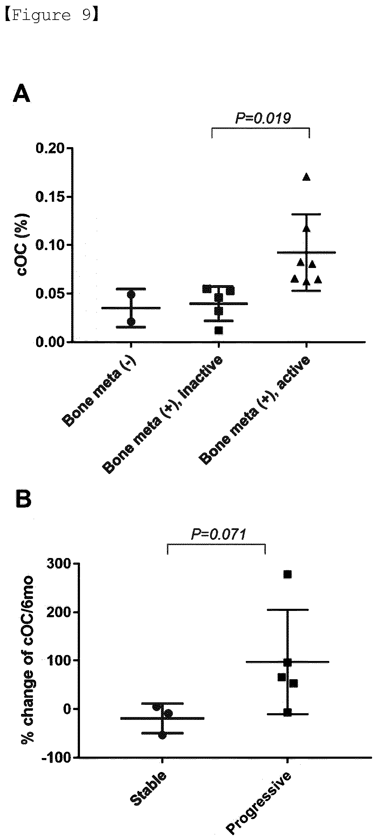

In order to evaluate whether circulating osteocalcin-positive cells present in the blood of actual patients with metastatic bone cancer have clinical diagnostic value by analyzing the circulating osteocalcin-positive cells, the number (%) of circulating osteocalcin-positive cells was measured on 14 thyroid cancer patients with metastatic bone cancer. Patient's blood sampling, mononuclear cell isolation and cell staining processes were performed in the same manner as described in Example 1 above. At the time of cOC% analysis and after continuous follow-up, the patients were divided into an active group with progression of metastatic bone disease and an inactive group without progression of metastatic bone disease and analyzed. The results are shown in FIG. 9.

#### (2) Experimental Results

1. Among 14 metastatic thyroid cancer patients, 12 patients with bone metastasis were divided into an inactive group of 5 patients and an active group of 7 patients depending on whether the metastatic bone disease was active or inactive at the time of cOC% analysis, and as a result, the cOC% of the active group was significantly higher than that of the inactive group (FIG. 9 (A)).

2. When the patients were divided into a stable group without progression of metastatic bone disease and a progressive group with progression of metastatic bone disease after 6-month follow-up, the baseline cOC% of the progressive group was higher than that of the stable group, but this difference was not statistically significant (FIG. 9(B)).

### Claims

1.  In vitro use of a composition comprising an agent for detecting cells released into blood expressing osteocalcin for diagnosing bone metastasis of cancer.

2.  The use of claim 1, wherein the composition further comprises an agent for detecting cells released into blood expressing N-cadherin.

3.  The use of claim 1, wherein the agent for detecting cells released into blood expressing osteocalcin is at least one selected from the group consisting of antibodies, aptamers, DNA, RNA, proteins, and polypeptides.

4.  The use of claim 2, wherein the agent for detecting cells released into blood expressing osteocalcin and the agent for detecting blood-derived cells expressing N-cadherin are each independently at least one selected from the group consisting of antibodies, aptamers, DNA, RNA, proteins, and polypeptides.

5.  The use of claim 1, wherein the cancer is at least one selected from among liver cancer, lung cancer, bladder cancer, stomach cancer, breast cancer, uterine cancer, colorectal cancer, colon cancer, blood cancer, ovarian cancer, prostate cancer, pancreatic cancer, spleen cancer, testicular cancer, thymus cancer, brain cancer, esophageal cancer, kidney cancer, biliary tract cancer, thyroid cancer, or skin cancer.

6.  The use of claim 1, wherein the cancer is breast cancer or thyroid cancer.

7.  In vitro use of a kit for diagnosing bone metastasis of cancer, the kit comprising the composition used according to any one of claims 1 to 6.

8. A method of providing information for diagnosis of bone metastasis of cancer, the method comprising a step of detecting cells released into blood expressing osteocalcin in a sample isolated from a subject to be diagnosed.

9. The method of claim 8, further comprising a step of detecting cells released into blood expressing N-cadherin in the sample.

10. The method of claim 8, wherein the cancer is at least one selected from among liver cancer, lung cancer, bladder cancer, stomach cancer, breast cancer, uterine cancer, colorectal cancer, colon cancer, blood cancer, ovarian cancer, prostate cancer, pancreatic cancer, spleen cancer, testicular cancer, thymus cancer, brain cancer, esophageal cancer, kidney cancer, biliary tract cancer, thyroid cancer, or skin cancer.

11. The method of claim 8, wherein the cancer is breast cancer or thyroid cancer.

**Patentansprüche**

1. In-vitro-Verwendung einer Zusammensetzung, die ein Mittel zum Nachweis von in Blut freigesetzten Zellen, die Osteocalcin exprimieren, enthält, zur Diagnose von Knochenmetastasen bei Krebs.

2. Verwendung nach Anspruch 1, wobei die Zusammensetzung weiter ein Mittel zum Nachweis von in das Blut freigesetzten Zellen, die N-Cadherin exprimieren, umfasst.

3. Verwendung nach Anspruch 1, wobei das Mittel zum Nachweis von in das Blut freigesetzten Zellen, die Osteocalcin exprimieren, mindestens eines ist, das aus der Gruppe ausgewählt ist, die aus Antikörpern, Aptameren, DNA, RNA, Proteinen und Polypeptiden besteht.

4. Verwendung nach Anspruch 2, wobei das Mittel zum Nachweis von in das Blut freigesetzten Zellen, die Osteocalcin exprimieren, und das Mittel zum Nachweis von aus dem Blut stammenden Zellen, die N-Cadherin exprimieren, jeweils unabhängig voneinander mindestens eines sind, das aus der Gruppe ausgewählt ist, die aus Antikörpern, Aptameren, DNA, RNA, Proteinen und Polypeptiden besteht.

5. Verwendung nach Anspruch 1, wobei der Krebs mindestens einer ist, ausgewählt aus Leberkrebs, Lungenkrebs, Blasenkrebs, Magenkrebs, Brustkrebs, Gebärmutterkrebs, Kolorektalkrebs, Kolonkrebs, Blutkrebs, Eierstockkrebs, Prostatakrebs, Bauchspeicheldrüsenkrebs, Milzkrebs, Hodenkrebs, Thymuskrebs, Gehirnkrebs, Speiseröhrenkrebs, Nierenkrebs, Gallengangkrebs, Schilddrüsenkrebs oder Hautkrebs.

6. Verwendung nach Anspruch 1, wobei der Krebs Brustkrebs oder Schilddrüsenkrebs ist.

7. In-vitro-Verwendung eines Kits zur Diagnose von Knochenmetastasen bei Krebs, wobei das Kit die nach einem der Ansprüche 1 bis 6 verwendete Zusammensetzung umfasst.

8. Verfahren zur Bereitstellung von Informationen für die Diagnose von Knochenmetastasen bei Krebs, wobei das Verfahren einen Schritt des Nachweises von Zellen, die in das Blut freigesetzt wurden und die Osteocalcin exprimieren, in einer aus einem zu diagnostizierenden Subjekt isolierten Probe umfasst.

9. Verfahren nach Anspruch 8, das weiter einen Schritt zum Nachweis von in das Blut freigesetzten Zellen, die N-Cadherin exprimieren, in der Probe umfasst.

10. Verfahren nach Anspruch 8, wobei der Krebs mindestens einer ist, ausgewählt aus Leberkrebs, Lungenkrebs, Blasenkrebs, Magenkrebs, Brustkrebs, Gebärmutterkrebs, Kolorektalkrebs, Kolonkrebs, Blutkrebs, Eierstockkrebs, Prostatakrebs, Bauchspeicheldrüsenkrebs, Milzkrebs, Hodenkrebs, Thymuskrebs, Gehirnkrebs, Speiseröhrenkrebs, Nierenkrebs, Gallengangkrebs, Schilddrüsenkrebs oder Hautkrebs.

11. Verfahren nach Anspruch 8, wobei der Krebs Brustkrebs oder Schilddrüsenkrebs ist.

**Revendications**

1. Utilisation in vitro d'une composition comprenant un agent de détection de cellules libérées dans le sang exprimant l'ostéocalcine pour le diagnostic de métastases osseuses du cancer.

2. Utilisation selon la revendication 1, dans laquelle la composition comprend en outre un agent de détection de cellules libérées dans le sang exprimant la N-cadhérine.

3. Utilisation selon la revendication 1, dans laquelle l'agent de détection de cellules libérées dans le sang exprimant l'ostéocalcine est au moins une choisie dans le groupe constitué par des anticorps, aptamères, ADN, ARN, protéines et polypeptides.

4. Utilisation selon la revendication 2, dans laquelle l'agent de détection de cellules libérées dans le sang exprimant l'ostéocalcine et l'agent de détection des cellules dérivées du sang exprimant la N-cadhérine sont chacun indépendamment au moins un choisi dans le groupe constitué par des anticorps, aptamères, ADN, ARN, protéines et polypeptides.

5. Utilisation selon la revendication 1, dans laquelle le cancer est au moins un cancer choisi parmi le cancer du foie, cancer du poumon, cancer de la vessie, cancer de l'estomac, cancer du sein, cancer de l'utérus, cancer colorectal, cancer du côlon, cancer du sang, cancer de l'ovaire, cancer de la prostate, cancer du pancréas, cancer de la rate, cancer du testicule, cancer du thymus, cancer du cerveau, cancer de l'œsophage, cancer du rein, cancer du tractus biliaire, cancer de la thyroïde, ou cancer de la peau.

6. Utilisation selon la revendication 1, dans laquelle le cancer est un cancer du sein ou un cancer de la thyroïde.

7. Utilisation in vitro d'un kit permettant de diagnostiquer des métastases osseuses d'un cancer, le kit comprenant la composition utilisée selon l'une quelconque des revendications 1 à 6.

8. Procédé de fourniture d'informations pour un diagnostic de métastases osseuses du cancer, le procédé comprenant une étape de détection de cellules libérées dans le sang exprimant l'ostéocalcine dans un échantillon isolé d'un sujet à diagnostiquer.

9. Procédé selon la revendication 8, comprenant en outre une étape de détection de cellules libérées dans le sang exprimant la N-cadhérine dans l'échantillon.

10. Procédé selon la revendication 8, dans lequel le cancer est au moins un cancer choisi parmi le cancer du foie, cancer du poumon, cancer de la vessie, cancer de l'estomac, cancer du sein, cancer de l'utérus, cancer colorectal, cancer du côlon, cancer du sang, cancer de l'ovaire, cancer de la prostate, cancer du pancréas, cancer de la rate, cancer du testicule, cancer du thymus, cancer du cerveau, cancer de l'œsophage, cancer du rein, cancer du tractus biliaire, cancer de la thyroïde, ou cancer de la peau.

11. Procédé selon la revendication 8, dans lequel le cancer est un cancer du sein ou un cancer de la thyroïde.

【Figure 1】

【Figure 2】

【Figure 3】

**(a)**

**(b)**

【Figure 4】

【Figure 5】

【Figure 6】

【Figure 7】

**A**  Inactive Disease at Baseline

**B**  Active Disease at Baseline

【Figure 8】

**(a)**

**Cut-off : 0.45%**

**(b)**

| Groups | n | Median PFS (months) |
|---|---|---|
| cOC < 0.045% | 35 | Not reached |
| cOC ≥ 0.045% | 28 | 12.0 |

HR=4.79 (95% CI=2.17~10.56)
*P<.001*

| | 0 | 5 | 10 | 15 | Months |
|---|---|---|---|---|---|
| cOC < 0.45% | 28 | 28 | 26 | 24 | |
| cOC ≥ 0.45% | 35 | 25 | 20 | 13 | *(n)* |

【Figure 9】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DAVIES S R et al.** OSTEOCALCIN AS A PROGNOSTIC INDICATOR FOR BONE METASTASIS IN DUCTAL BREAST CANCER. *CALCIFIED TISSUE INTERNATIONAL,* 01 July 2008, vol. 83 (1), 27-27
- Predictive value of osteocalcin in bone metastatic differentiated thyroid carcinoma. **GAO Y et al.** CLINICAL BIOCHEMISTRY. ELSEVIER INC, 01 February 2010, vol. 43, 291-295
- Physiological strains remodel extracellular matrix and cell-cell adhesion in osteoblastic cells cultured on alumina-coated titanium alloy. **DI PALMA F et al.** BIOMATERIALS. ELSEVIER, 01 June 2004, vol. 25, 2565-2575
- In Situ Visualization of Human MSC Engraftment in Bone Marrow: Integration into Murine Microenvironment and Interaction with Human HSC. **MUGURUMA YUKARI et al.** BLOOD. AMERICAN SOCIETY OF HEMATOLOGY, 16 November 2004, vol. 104, 1283